# FASCICULE DE BREVET EUROPEEN

(11) **EP 4 373 415 B1**
(45) Date de publication et mention de la délivrance du brevet: **22.10.2025**
(21) Numéro de dépôt: 22754485.5
(22) Date de dépôt: 19.07.2022
(51) Int. Cl.: A61B 17/20, A61B 10/00, A61M 5/32, A61B 5/00

(54) **APPAREIL POUR INJECTION DANS LA PEAU PAR INCISION SUPERFICIELLE ET SON ENSEMBLE DE CHARGEMENT**
VORRICHTUNG ZUR INJEKTION IN DIE HAUT DURCH OBERFLÄCHLICHEN EINSCHNITT UND LADEANORDNUNG DAFÜR
APPARATUS FOR INJECTION IN THE SKIN BY SUPERFICIAL INCISION, AND ITS LOADING ASSEMBLY

(30) Priorité: 20.07.2021 FR 2107823
(43) Date de publication de la demande: 29.05.2024
(73) Titulaire: A2M, 85700 Saint-Mesmin (FR)
(72) Inventeur: SOURISSEAU, Thierry, 85700 Saint-Mesmin (FR)
(74) Mandataire: Regimbeau
(86) Numéro de dépôt international: PCT/FR2022/051434
(87) Numéro de publication internationale: WO 2023/002118

(56) Documents cités:
- GB-A- 2 450 152
- US-A- 5 139 029
- US-A- 6 024 706
- US-A1- 2014 276 196
- US-A1- 2018 344 999

## Description

### DOMAINE DE L'INVENTION

L'invention concerne le domaine des dispositifs pour injection dans la peau par incision superficielle et leur chargement. En particulier, l'invention concerne les dispositifs de réalisation de diagnostic allergique ou les dispositifs de vaccination par incision de la peau d'un patient et les appareils et procédés pour porter simultanément plusieurs dispositifs de ce type afin de réaliser simultanément plusieurs diagnostics allergiques ou plusieurs vaccinations.

### ETAT DE LA TECHNIQUE

Les dispositifs pour injection dans la peau par incision superficielle peuvent servir aux tests de diagnostics allergiques ou aux vaccinations.

Les tests de diagnostic sont des examens médicaux consistant à faire pénétrer superficiellement quelques micro-gouttes d'un produit (allergène par exemple) dans la partie supérieure de l'épiderme d'un patient ou d'un animal, afin de tester la réactivité audit produit. Ces tests de diagnostics allergiques cutanés sont généralement dénommés « prick test ».

En pratique, après avoir déposé une goutte de produit sur la peau, on pique la peau à travers la goutte avec une pointe pour faire pénétrer du produit sous la peau, puis on attend quinze à vingt minutes avant d'interpréter la réaction cutanée.

Il existe sur le marché des dispositifs d'incision qui présentent une pointe et dont la pointe contient un faible volume de produit réactif. Un tel dispositif d'incision est configuré pout pratiquer une incision de la peau et simultanément faire pénétrer le produit réactif dans la peau.

Il existe sur le marché des dispositifs d'incision contenant eux une solution vaccinale, le dispositif d'incision étant capable d'inciser de la peau et simultanément de faire pénétrer le vaccin dans la peau.

Des appareils destinés à effectuer plusieurs tests ou plusieurs vaccins simultanément existent. Un tel appareil peut être conçu comme un réceptacle de plusieurs dispositifs d'incision, c'est-à-dire qu'il est configuré pour recevoir et porter plusieurs dispositifs d'incision. Chaque dispositif est par exemple placé de sorte que sa pointe fait saillie d'une surface de l'appareil. L'appareil est ensuite mis en contact contre la peau du patient de sorte à piquer la peau avec chacune des pointes des dispositifs qui fait pénétrer le produit utile dans la peau. Les appareils existants ne permettent cependant par une application des pointes de sorte que chacun des dispositifs fait pénétrer effectivement le produit utile dans l'épiderme. Cela diminue l'intérêt d'un tel appareil qui n'assure pas toutes les tâches médicales (test diagnostique ou vaccination) qu'il est supposé remplir de manière simultanée.

Il existe donc un besoin d'un appareil permettant d'effectuer en une seule application une pluralité d'incisions cutanées pour faire pénétrer un produit utile, et de réaliser plus efficacement une pluralité de tâches médicales comme une pluralité de tests diagnostiques ou une pluralité de vaccinations.

Le document US2014276196A1 est considéré comme l'art antérieur le plus proche et présente un dispositif diagnostic allergique et le document US2018344999A1 présente des patchs à micro-aiguilles pour administration transdermique.

### EXPOSE DE L'INVENTION

Un but de l'invention est de proposer un appareil pour effectuer simultanément une pluralité d'incisions cutanées de sorte à faire pénétrer un produit utile dans la peau par chaque incision, réaliser ainsi en une seule application et de manière plus efficace que dans l'art antérieur une pluralité de tâches médicales comme une pluralité de tests diagnostiques ou une pluralité de vaccinations.

Le but est atteint dans le cadre de la présente invention grâce à un appareil pour injection dans la peau par incision superficielle comprenant un corps principal, et des pointes pour incision de la peau s'étendant en saillie du corps, l'appareil étant apte à avoir une configuration dans laquelle les pointes définissent une surface intégralement courbe, la surface étant convexe dans une direction longitudinale, et un centre de courbure d'une section de la surface dans un plan longitudinal parallèle à la direction longitudinale et le corps sont d'un même côté de la surface.

Une telle disposition des pointes permet de faire rouler l'appareil sur la peau selon cette surface dans la direction longitudinale, de sorte que chacune des pointes fait pénétrer effectivement du produit utile dans l'épiderme.

Le caractère courbe de la surface assure la continuité de la surface et permet l'absence d'arête séparant une première zone d'une deuxième zone de l'appareil. Cela permet la continuité du geste d'application : l'opérateur n'ayant pas à gérer le passage de la première zone à la deuxième, il n'a pas à fournir un effort particulier dans l'application pour produire un appui identique d'une zone à une autre.

L'appareil est avantageusement et optionnellement complété par les différentes caractéristiques suivantes prises seules ou en combinaison :
- le corps principal comprend une face, les pointes s'étendant en saillie de la face, la face définissant lorsque l'appareil est dans la configuration un rayon de courbure moyen dans le plan longitudinal, le rayon de courbure moyen étant compris entre 7 et 20 centimètres ;
- deux branches disposées à deux extrémités longitudinales du corps principal, les deux branches étant directement liées l'une à l'autre lorsque l'appareil est dans la configuration de sorte à former une poignée, le corps principal et la poignée étant d'un même côté de la surface ;
- la poignée présente une forme cylindrique définie par la direction axiale perpendiculaire à la direction longitudinale ;
- lorsque l'appareil est dans la configuration, la surface et la face sont concaves dans une direction axiale perpendiculaire à la direction longitudinale, la surface étant située entre un centre de courbure d'une section de la surface dans un plan axial parallèle à la direction axiale et le corps ;
- la face est cylindrique, la face cylindrique étant définie par une direction axiale perpendiculaire à la direction longitudinale ;
- le corps principal comprend des éléments liés deux à deux et juxtaposés dans un plan normal à la direction axiale, deux des éléments étant liés chacun à une des branches, chaque liaison entre deux éléments adjacents ou entre un élément et une branche permettant une rotation autour d'un axe parallèle à la direction axiale ;
- chaque élément présente un flanc en regard de chaque élément adjacent ou d'une branche adjacente, chaque branche présente un flanc en regard d'un élément adjacent, de sorte que les flancs de deux éléments adjacents ou d'un élément et d'une branche adjacents sont complémentaires et, lorsque l'appareil est dans la configuration en contact l'un avec l'autre ;
- les flancs définissent, lorsque l'appareil est dans la configuration, un plan passant par un centre de courbure de la surface et par un axe de la liaison liant les deux éléments ou l'élément et la branche ;
- des dispositifs logés dans des ouvertures du corps principal et portant les pointes respectives, chaque ouverture traversant le corps principal, chaque élément comprenant une des ouvertures et un des dispositifs, les éléments présentant une forme identique et pour chaque couple d'éléments adjacents les éléments présentant des sens d'orientation opposés ;
- chaque élément comprend :
   - un butoir configuré pour couvrir, lorsque l'appareil est dans la configuration, une tête d'un premier dispositif d'un premier élément adjacent, le butoir étant en contact avec le premier dispositif, et/ou
   - une paroi de butée configurée pour, lorsque l'appareil est dans la configuration, être en contact avec une tête d'un deuxième dispositif d'un deuxième élément adjacent différent du premier élément adjacent ;
- des dispositifs logés dans des ouvertures du corps et portant les pointes respectives, chaque ouverture traversant le corps principal, la configuration étant une configuration d'incision, l'appareil étant apte à avoir une configuration de retrait dans laquelle chaque ouverture est découverte de sorte à permettre un retrait d'un dispositif.

L'invention porte également sur un ensemble de chargement comprenant un appareil tel qu'on vient de le décrire, et un dévidoir, le dévidoir comprenant un logement et une lamelle agencée au fond du logement, le logement présentant des cavités et la lamelle comprenant des trous, le corps principal comprenant des reliefs de centrage de forme complémentaire aux cavités et disposés le long de bords latéraux du corps principal, le logement étant configuré pour recevoir le corps principal de l'appareil de sorte que les reliefs s'insèrent dans les cavités, et que les trous reçoivent les pointes.

Un tel ensemble est avantageusement et optionnellement complété par les caractéristiques suivantes prises seules ou en combinaison :
- l'appareil comprenant des dispositifs logés dans des ouvertures du corps et portant les pointes respectives, chaque ouverture traversant le corps principal, la configuration étant une configuration d'incision, l'appareil étant apte à avoir une configuration de retrait dans laquelle chaque ouverture est découverte de sorte à permettre un retrait d'un dispositif, l'ensemble comprenant une tête, la tête comprenant des canaux configurés pour permettre un glissement d'un dispositif d'incision, chaque canal traversant la tête depuis une entrée évasée dans une face supérieure de la tête jusqu'à une sortie dans une face inférieure de la tête, la tête étant configurée pour couvrir le corps principal de l'appareil lorsque le corps principal en configuration de retrait est reçu dans le logement, la face inférieure étant en appui sur des parois latérales du dévidoir, chaque canal de chargement présentant une sortie en regard d'une ouverture de sorte qu'un dispositif glissant dans le canal passe dans l'ouverture ;
- les canaux présentent une forme d'hélice autour d'une direction d'extension du canal, de sorte que le glissement d'un dispositif d'incision depuis la face supérieure jusqu'à la face inférieure fait tourner le dispositif d'un angle de 90° autour de la direction d'extension.

En outre l'invention porte sur un procédé d'incision superficielle cutanée dans lequel on fait rouler sur la peau d'un patient un appareil portant des pointes d'incision pour diagnostic allergique en mettant les pointes successivement en contact avec la peau.

Le procédé d'incision peut avantageusement et optionnellement être complété par la caractéristique suivante : l'appareil est un appareil tel qu'on l'a décrit précédemment, le procédé comprenant une étape d'adaptation de l'appareil dans la configuration d'incision avant de le faire rouler sur la peau.

Enfin l'invention porte sur un procédé de préparation de l'appareil d'un ensemble comme décrit ci-avant, le procédé comprenant les étapes suivantes :
- nettoyage de la lamelle,
- versement d'un produit utile dans un trou de la lamelle,
- réception de l'appareil dans le logement.

Le procédé de préparation peut avantageusement et optionnellement être complété par une étape de couverture du corps principal reçu dans le logement par la tête de chargement et une étape d'insertion d'un dispositif d'incision vide de produit utile dans une entrée d'un canal de chargement.

### DESCRIPTION DES FIGURES

D'autres caractéristiques et avantages de l'invention ressortiront encore de la description qui suit, laquelle est purement illustrative et non limitative, et doit être lue en regard des dessins annexés sur lesquels :
[Fig. 1]
[Fig. 2]
[Fig. 3] Les figures 1, 2 et 3 illustrent schématiquement un appareil pour injection dans la peau par incision superficielle selon un mode de réalisation de l'invention,
[Fig. 4] La figure 4 illustre schématiquement un dispositif d'incision cutanée,
[Fig. 5] La figure 5 illustre schématiquement un corps principal d'un appareil pour injection dans la peau par incision superficielle selon un mode de réalisation de l'invention.
[Fig. 6] La figure 6 représente schématiquement un élément du corps principal selon un mode de réalisation de l'invention.
[Fig. 7] La figure 7 illustre schématiquement un corps principal d'un appareil pour injection dans la peau par incision superficielle selon un mode de réalisation de l'invention.
[Fig. 8] La figure 8 représente schématiquement un élément du corps principal selon un mode de réalisation de l'invention.
[Fig. 9] La figure 9 représente de manière schématique un dévidoir.
[Fig. 10]

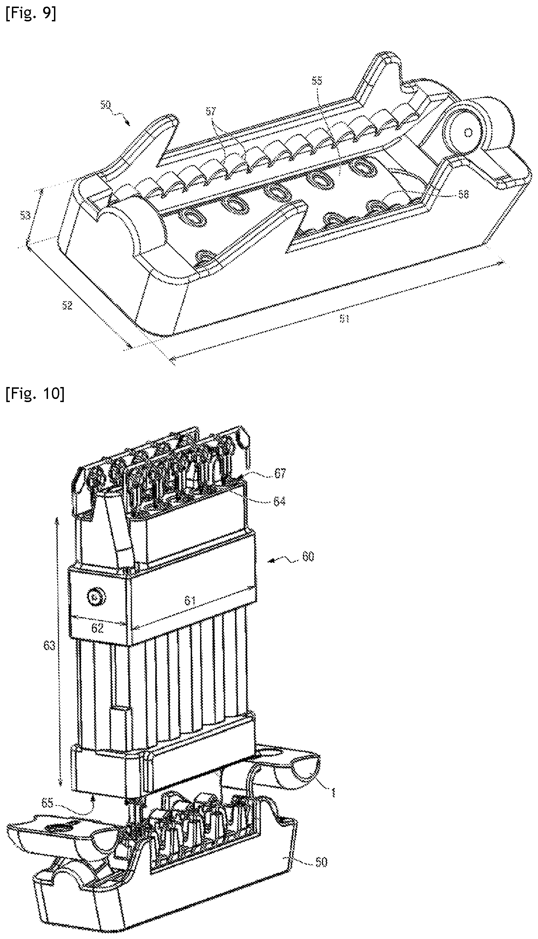

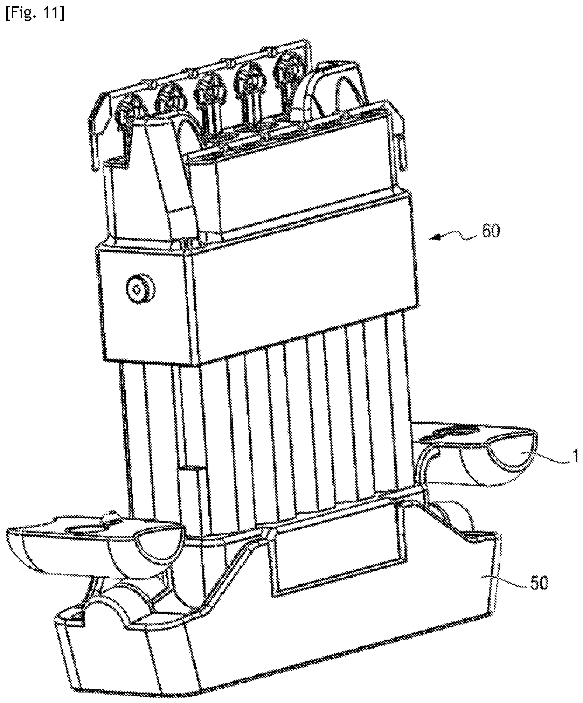

[Fig. 11] Les figures 10 et 11 illustrent schématiquement un mode de réalisation d'un ensemble de chargement.

### DESCRIPTION DETAILLEE DE L'INVENTION

### Appareil pour injection dans la peau

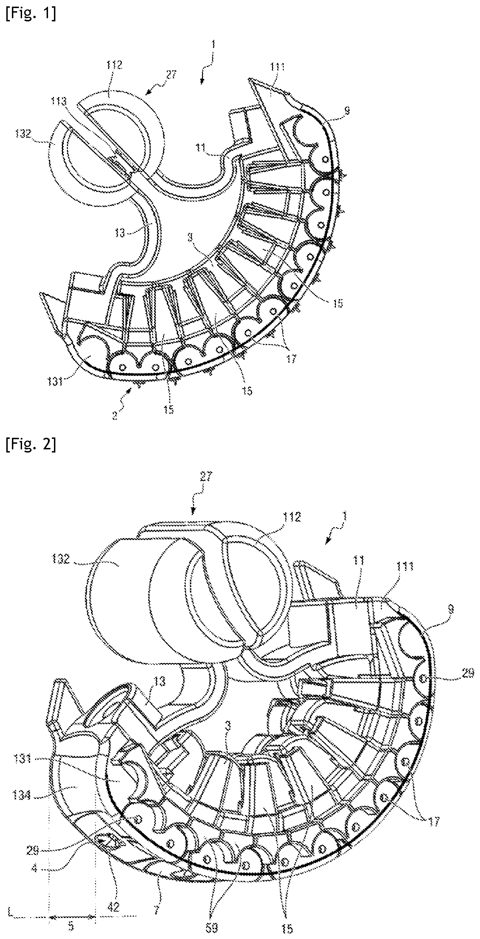

Les figures 1, 2 et 3 illustrent schématiquement un appareil selon un premier mode de réalisation de l'invention.

L'appareil 1 comprend des dispositifs d'incision cutanée 2 destinés à faire pénétrer un produit utile dans la peau comme un réactif allergène ou respectivement une solution vaccinale afin de tester la réactivité de la personne ou de l'animal au réactif ou respectivement vacciner le patient ou l'animal.

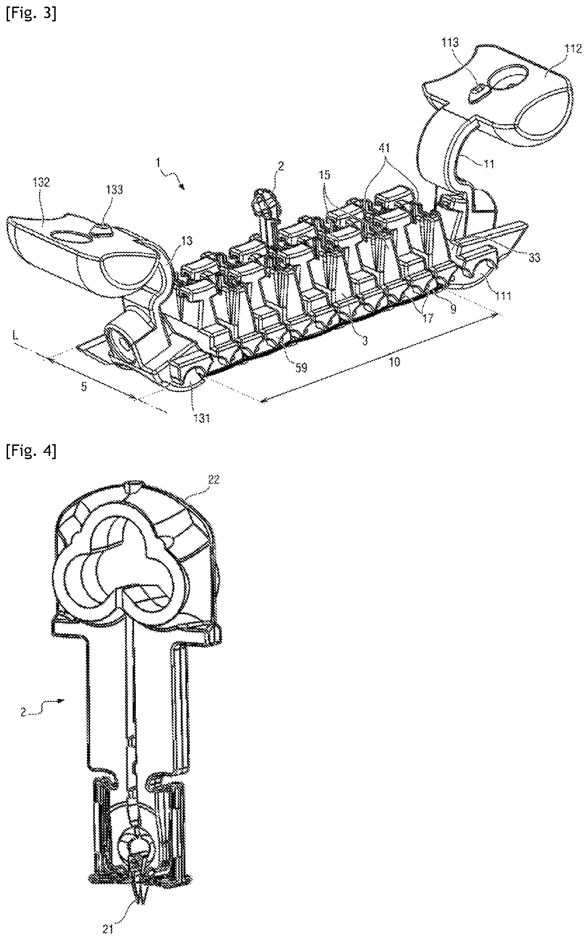

Les dispositifs 2 d'incision cutanée peuvent notamment être les dispositifs décrits dans la demande FR3070013. La figure 4 illustre schématiquement un dispositif 2 d'incision cutanée. Chaque dispositif 2 comprend à une première extrémité une pointe 21 d'incision de la peau (pour diagnostic allergique ou pour vaccination), et à une deuxième extrémité opposée à la première extrémité une tête 22.

L'appareil 1 comprend un corps principal 3 qui s'étend sur une largeur 5 selon une première direction L dite direction latérale ou direction axiale.

La largeur 5 est typiquement comprise entre 2 et 30 cm.

Le corps principal 3 est configuré pour fixer des dispositifs 2 d'incision cutanée.

La fixation des dispositifs dans le corps principal est configurée de sorte que les pointes s'étendent en saillie du corps principal (3).

Les dispositifs peuvent être identiques ou différents

### Surface des pointes dans la configuration d'incision

L'appareil comprend le corps principal et les pointes d'incision de la peau. Il peut être agencé dans une configuration d'incision dans laquelle les pointes définissent une surface intégralement courbe, la surface étant convexe dans une direction longitudinale, et un centre de courbure d'une section de la surface dans un plan longitudinal parallèle à la direction longitudinale et le corps sont d'un même côté de la surface.

La configuration d'incision est représentée aux figures 1 et 2.

La surface en question est virtuelle et passe par l'extrémité des pointes.

Cette surface, dite surface des pointes, est intégralement courbe, c'est-à-dire que la surface n'est pas plane et ne présente pas d'arêtes. Autrement dit la surface n'est pas une surface plane ou une pluralité de surfaces qui se joignent le long d'arêtes.

La surface des pointes est convexe dans une direction longitudinale, autrement dit toute section de la surface des pointes dans un plan longitudinal parallèle à la direction longitudinale présente une courbure définie par un ou plusieurs centres de courbures, le ou les centres de courbures étant du côté du corps principal. Autrement dit chaque dispositif qui a sa pointe sur la surface est du même côté de la surface que le ou les centres de courbures de la section de la surface des pointes dans un plan longitudinal.

L'utilisation de l'appareil décrit plus haut permet de mettre en œuvre un procédé d'incision superficielle cutanée dans lequel on fait rouler sur la peau d'un patient l'appareil portant des pointes 21 d'incision de la peau selon dans la direction longitudinale, ce qui permet de mettre les pointes successivement en contact avec la peau.

En option la surface des pointes peut être concave dans une direction axiale perpendiculaire à la direction longitudinale. La surface des pointes peut ainsi être roulée dans la direction longitudinale et présenter une forme creuse dans la direction axiale. La forme creuse peut notamment être adaptée à une forme d'une partie du corps sur laquelle l'appareil est mis en mouvement, comme un bras ou un avant-bras par exemple.

En option, la surface des pointes peut être cylindrique c'est-à-dire qu'elle est définie par un axe et une courbe génératrice. La surface cylindrique est déterminée par des droites parallèles à l'axe, les droites s'appuyant sur la courbe génératrice. L'intersection de la surface cylindrique définit et d'un plan normal à l'axe est la courbe génératrice. La surface des pointes peut ainsi être roulée dans la direction longitudinale et présenter une forme plate dans la direction axiale. Cette forme plate est adaptée à une forme plate d'une partie du corps sur laquelle l'appareil est mis en mouvement, comme un dos par exemple.

Comme la surface cylindrique est intégralement courbe et convexe, sa courbe génératrice est intégralement courbe et convexe.

Lorsque la surface des pointes est cylindrique, son axe est la première direction L dite direction latérale ou direction axiale.

Dans la configuration d'incision selon cette même option, la courbe génératrice peut être un arc de cercle ou proche d'un arc de cercle.

Lorsque la courbe génératrice est un arc de cercle, les rayons de courbure de la courbe génératrice sont tous égaux à une valeur prédéterminée correspondant au rayon de l'arc de cercle.

Lorsque la courbe génératrice est proche d'un arc de cercle, le rapport de tout rayon de courbure de la courbe génératrice sur une valeur prédéterminée est compris entre 0,9 et 1,1.

La valeur prédéterminée des rayons de courbure peut être comprise entre 7 et 20 cm.

D'autres surfaces de pointes peuvent être envisagées pour la configuration d'incision comme des surfaces sphériques ou des surfaces paraboliques.

### Face du corps principal

Les pointes qui s'étendent en saillie du corps s'étendent en saillie d'une face 7 comprise dans le corps principal 3.

La face 7 s'étend dans le plan longitudinal sur une longueur finie 10. Typiquement la longueur 10 est comprise entre 5 cm et 30 cm.

Dans la configuration d'incision, la face 7 définit un rayon de courbure moyen dans le plan longitudinal, le rayon de courbure étant compris entre 7 et 20 centimètres.

Cette face peut être intégralement courbe, c'est-à-dire que la face n'est pas plane et ne présente pas d'arêtes.

La face des pointes peut être convexe dans la direction longitudinale, autrement dit toute section de la face dans un plan longitudinal présente une courbure définie par un ou plusieurs centres de courbures, le ou les centres de courbures étant du côté du corps principal.

La courbure de la face dans un plan longitudinal correspond à un rayon de courbure moyen comprise entre 7 et 20 cm.

Dans la configuration d'incision, lorsque la surface est concave dans la direction axiale, la face est également concave dans la direction axiale, la surface étant située entre un centre de courbure d'une section de la surface des pointes dans un plan axial parallèle à la direction axiale et le corps.

Dans la configuration d'incision, lorsque la surface est cylindrique définie par la direction axiale, la face est également cylindrique définie par la direction axiale. La face cylindrique est engendrée par des droites parallèles à la direction axiale L, les droites s'appuyant sur la courbe génératrice.

La courbe génératrice 9 est un segment courbe qui n'est pas fermé sur lui-même. La face cylindrique 7 s'étend dans la direction axiale L sur la largeur 5. Autrement dit, la face cylindrique 7 est obtenue en balayant la courbe génératrice 9 sur la largeur 5 selon la direction axiale L.

La courbe génératrice 9 est contenue dans un plan normal à la direction axiale L.

La face cylindrique 7 présente la forme d'un ruban, sa longueur 10 étant supérieure à sa largeur 5.

La courbe génératrice définit une direction longitudinale dans laquelle s'étend le corps principal. Les extrémités du corps principal dans cette direction sont appelées extrémités longitudinales.

Le corps principal 3 s'étend d'un côté de la face cylindrique, dit côté intérieur de la face cylindrique.

La courbe génératrice 9 peut prendre différentes formes, ce qui impose des formes différentes à la face cylindrique 7 et au corps principal 3.

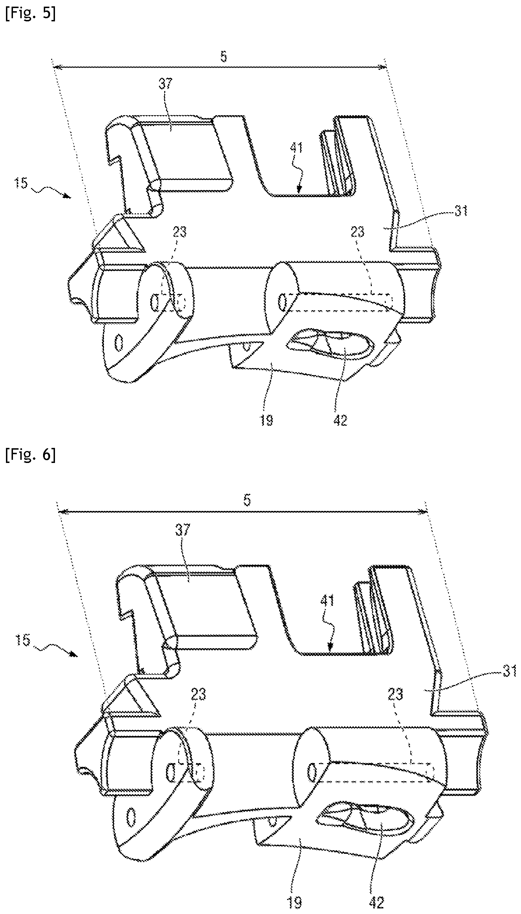

Dans cette option de face cylindrique, l'ouverture est alignée selon une direction perpendiculaire à la face cylindrique 7, comme représenté sur la figure 5 illustrant schématiquement un corps principal 3.

Dans la configuration d'incision lorsque la surface des pointes est cylindrique selon un arc de cercle ou proche d'un arc de cercle, la face cylindrique est également un arc de cercle ou proche d'un arc de cercle.

Lorsque la courbe génératrice est un arc de cercle, les rayons de courbure de la courbe génératrice sont tous égaux à une valeur prédéterminée correspondant au rayon de l'arc de cercle.

Lorsque la courbe génératrice est proche d'un arc de cercle, le rapport de tout rayon de courbure de la courbe génératrice sur une valeur prédéterminée est compris entre 0,9 et 1,1. La valeur prédéterminée correspond dans ce cas à un rayon de courbure moyen de la courbe génératrice dans le plan normal.

La face cylindrique 7 s'étend dans un plan normal à la direction axiale sur une longueur, la valeur prédéterminée (rayon de l'arc de cercle ou rayon de courbure moyen de la courbe génératrice) est supérieure à la longueur.

De manière avantageuse, la valeur prédéterminée (rayon de l'arc de cercle ou rayon de courbure moyen de la courbe génératrice) est cinq fois supérieure à la longueur.

Lorsque les rayons de courbure sont égaux à la valeur prédéterminée, la courbe génératrice est un arc de cercle qui s'étend sur un angle compris entre 10°et 150°. L'arc de cercle peut s'étendre préférentiellement sur un angle compris entre 20° et 140°.

La valeur prédéterminée (rayon de l'arc de cercle ou rayon de courbure moyen de la courbe génératrice) prend typiquement une valeur entre 7 cm et 20 cm. D'autres faces peuvent être envisagées pour la configuration d'incision comme des faces sphériques ou des faces paraboliques.

### Branches de l'appareil

Le corps principal 3 comprend deux branches 11, 13 disposées à deux extrémités longitudinales du corps principal 3. Plus précisément les deux branches 11 et 13 sont placées à deux extrémités de la courbe génératrice.

Chaque branche est une pièce rigide non déformable qui s'étend dans la direction L sur la largeur 5.

Chaque branche se trouve dans le prolongement du corps principal 3, de sorte que dans la direction axiale L, le corps principal comme les branches s'étendent sur la largeur 5.

Chaque branche s'étend, dans le prolongement de la courbe génératrice, d'une première extrémité 111 et 131 reliée au corps principal jusqu'à une deuxième extrémité 112 et 132.

Lorsque l'appareil comprend les deux branches 11,13, celles-ci sont directement liées l'une à l'autre lorsque l'appareil est dans la configuration d'incision. Elles forment une poignée (27), le corps principal (3) et la poignée (27) étant d'un même côté de la surface.

Les deuxièmes extrémités 112 et 132 comprennent des moyens de liaison 113 et 133 pour se lier entre elles, de sorte à lier directement les deux branches entre elles.

Les moyens de liaison sont des moyens de liaison amovible comme par exemple des aimants, des pièces en tissu auto-agrippant, des clips de fixation, etc...

Les branches peuvent donc être liées entre elles ou disjointes.

Lorsque les deux branches sont liées entre elles directement par leurs deuxièmes extrémités, les deuxièmes extrémités forment la poignée 27, c'est-à-dire que la forme des deuxièmes extrémités liées ensemble est facilement préhensible dans la main ou entre deux doigts comme le pouce et l'index.

En option, notamment, cette poignée 27 présente une forme cylindrique définie par un axe qui est la direction axiale ou axiale (L). Autrement dit, la poignée présente une forme générée par une surface sur laquelle s'appuyent des droites parallèles à la direction axiale (L). Cette surface peut notamment être un cercle fermé. Par ailleurs la poignée peut présenter une encoche pour faciliter l'ouverture de la poignée, c'est-à-dire faciliter la désolidarisation des deuxièmes extrémités.

Lorsque les rayons de courbure sont égaux à la valeur prédéterminée, un centre de la face cylindrique, le centre de l'arc de cercle et un centre de la poignée peuvent être alignés.

L''appareil est alors configuré pour que la poignée soit centrée au-dessus du côté intérieur de la face cylindrique, ce qui permet une manipulation plus simple et plus intuitive de l'appareil.

Chaque branche 11,13 est reliée au corps principal 3 de sorte qu'une face, dite face de prise 114 et 134, de chaque branche se situe dans le prolongement de la face cylindrique. Les branches s'étendent d'un côté de la face de prise, dit côté intérieur de la face de prise. Le côté intérieur de la face de prise est dans le prolongement du côté intérieur de la face cylindrique.

Chaque branche est reliée au corps principal 3 de sorte que chaque branche est reliée à un élément situé à une extrémité de la juxtaposition d'éléments. Chaque branche est donc en regard d'un élément, définissant un couple de branche et élément adjacents.

La liaison entre le corps principal et une branche est un liaison pivot 29 qui permet une rotation entre ces deux pièces selon la direction axiale L. L'axe de rotation de cette liaison est suffisamment proche de la face cylindrique et de la face de prise de sorte que la face de prise est dans le prolongement de la face cylindrique.

Dans un premier mode de réalisation de l'appareil, la liaison pivot entre le corps principal et une branche est réalisée de la manière suivante : la branche comprend un passage s'étendant dans la direction latérale, un arbre de rotation occupe ce passage et le passage traversant un élément du corps principal, l'élément étant adjacent à la branche. L'arbre passant à travers la branche et l'élément lie ces deux pièces et permet une rotation entre elles autour de l'arbre de rotation.

Dans le deuxième mode de réalisation de l'appareil, la liaison pivot entre le corps principal et une branche est réalisée de la manière suivante : une couche de matière relie d'une part la branche et d'autre part l'élément adjacent, la zone étant suffisamment fine dans les directions perpendiculaires à la direction latérale pour permettre une rotation entre ces deux pièces entre ces deux éléments, autour de la couche.

Optionnellement, les deux branches peuvent présenter une forme identique, ce qui permet de limiter les couts de fabrication. Cette option est compatible avec des éléments identiques et en nombre pair.

### Eléments du corps principal

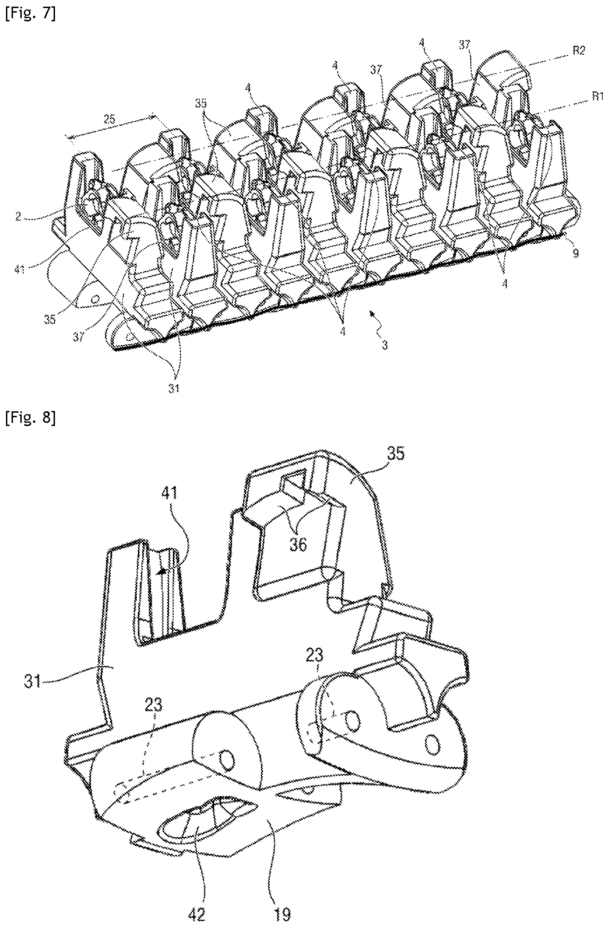

Le corps principal 3 comprend des éléments 15 s'étendant sur la largeur 5 du corps principal selon la direction latérale L. Les figures 6 et 8 représentent schématiquement un élément 15 compris dans le corps principal, selon un mode de réalisation particulier de l'appareil.

Selon ce mode particulier, le corps principal 3 comprend la face cylindrique 7 définie par une direction axiale L, les pointes 21 s'étendant en saillie de la face 7, le corps principal 3 comprenant des éléments 15 liés deux à deux et juxtaposés dans un plan normal à la direction axiale L, deux des éléments 15 étant liés chacun à une branche 11, 13, chaque liaison 17,29 entre deux éléments 15 adjacents ou entre un élément 15 et une branche 11, 13 permettant une rotation autour d'un axe parallèle à la direction axiale L.

Dans le plan perpendiculaire à la direction latérale L, les éléments 15 s'étendent depuis la face cylindrique 7 vers le côté intérieur. La face cylindrique comprend des faces extérieures 19 des éléments 15.

Dans ce même plan perpendiculaire, les éléments 15 sont juxtaposés les uns aux autres selon la courbe génératrice 9. Les éléments adjacents sont liés par une liaison pivot 17 permettant une rotation autour de la direction latérale L.

Les axes de ces liaisons pivot 17 sont placés proches de la face cylindrique, côté intérieur, de sorte que la face extérieure d'un élément est toujours en contact avec la face extérieure d'un élément adjacent.

La juxtaposition des éléments qui sont ainsi articulés permet de conférer un caractère déformable à la courbe génératrice 9 et au corps principal 3.

Dans le premier mode de réalisation de l'appareil déjà évoqué plus haut, les liaisons pivot 17 sont toutes réalisées de la manière suivante : chaque élément 15 comprend un passage 23 s'étendant selon la direction latérale L et pour chaque couple d'éléments adjacents un arbre de rotation occupe un passage de chacun des éléments 15 du couple, de sorte à passer à travers chaque élément du couple, à lier ces deux éléments et permettre une rotation entre eux autour de l'arbre de rotation.

Les passages dans les éléments sont placés proches de la face cylindrique, côté intérieur.

Dans le deuxième mode de réalisation déjà évoqué plus haut, les liaisons pivot sont toutes réalisées de la manière suivante : une couche de matière reliant des éléments 15 adjacents. La couche de matière s'étend dans la direction latérale et forme la seule liaison entre les éléments. La couche est choisie suffisamment fine dans les directions perpendiculaires à la direction latérale pour permettre une rotation autour de la direction latérale entre ces deux éléments autour de la couche. Optionnellement à ce deuxième mode de réalisation, la liaison peut être réalisée par plusieurs couches de matières dans le prolongement les unes des autres, selon la direction latérale.

Les couches dans les éléments sont placées proches de la face cylindrique, côté intérieur.

Du fait du caractère déformable de l'appareil 1 produit par les différentes liaisons pivot dans le corps principal 3 ou entre le corps principal 3 et chacune des branches 11, 13, l'appareil 1 est apte à avoir plusieurs configurations.

En particulier, l'appareil peut adapter la configuration d'incision déjà présentée plus haut.

### Surfaces des éléments et des branches en contact

Quel que soit le mode de réalisation des liaisons pivots, l'appareil peut dans une première option dite option de flancs complémentaires, présenter l'ensemble des caractéristiques suivantes : chaque élément présente une surface ou un flanc en regard de chaque élément adjacent ou d'une branche adjacente, chaque branche présente une surface ou un flanc 31 en regard d'un élément adjacent, de sorte que les surfaces ou les flancs de deux éléments adjacents ou d'un élément et d'une branche adjacents sont complémentaires. Une telle surface est illustrée en figures 6 et 7.

Dans cette option, chaque élément comprend deux surfaces qui s'étendent sur la largeur 5 de l'élément. Chaque surface est associée à une liaison pivot de l'élément, de sorte que l'axe de la liaison pivot se situe entre la face extérieure et la surface.

Si l'élément est en extrémité de la juxtaposition des éléments, alors cet élément comprend une surface qui fait face à une branche et une surface qui face à un autre élément. Sinon, chacune des deux surfaces de l'élément fait face à un autre élément.

Dans cette première option, chaque branche comprend une surface ou un flanc 33 qui s'étend sur la largeur 5 de la branche. La surface 33 est associée à la liaison pivot de la branche avec l'élément adjacent, de sorte que l'axe de la liaison pivot se situe entre la face de prise et la surface. La surface de la branche fait face à l'élément adjacent de la branche.

Dans tous les cas, chaque surface 31, 33 est en regard d'une autre surface, et elles sont complémentaires l'une de l'autre. Les liaisons pivot sont configurées pour permettre le contact entre deux surfaces en regard l'une de l'autre. Plus généralement, l'appareil est configuré pour permettre le contact simultané de tous les couples de surfaces en regard l'une de l'autre.

Dans un cas particulier les surfaces sont toutes planes. Chaque surface définit alors un plan passant par l'axe de la liaison pivot à laquelle la surface est associée.

Dans le cas de cette première option dite des flancs complémentaires, la configuration d'incision correspond à la situation où les surfaces situées en regard l'une de l'autre, telle qu'on les a définies plus haut, se trouvent en contact. Le contact entre les surfaces en regard empêche un sens de la rotation de la liaison pivot correspondant. Aussi, dans la configuration d'incision toutes les rotations sont bloquées dans un sens. Le fait de fermer la poignée en liant les deuxièmes extrémités des branches vient empêcher l'autre sens de la rotation, de sorte que l'appareil est stable dans la configuration d'incision. Il est possible d'ajuster la liaison entre les deuxièmes extrémités de sorte que l'appareil présente peu de jeu mécanique dans la configuration d'incision. Cela permet de stabiliser l'appareil dans sa configuration d'incision.

### Ouvertures

Les dispositifs 2 peuvent être fixés dans le corps principal 3 en y étant logés, le corps comprenant alors des ouvertures 4 pour accueillir et porter les dispositifs 2. Chaque ouverture 4 traverse le corps principal.

D'un côté du corps principal, l'ouverture comprend une entrée 41 par laquelle on fait entrer un dispositif d'incision cutanée.

L'ouverture comprend une butée contre laquelle le dispositif d'incision cutanée peut être maintenu.

De l'autre côté du corps principal, l'ouverture comprend une sortie 42 à travers laquelle s'étend une pointe du dispositif, lorsque celui-ci est en appui contre la butée.

Lorsqu'un dispositif d'incision 2 est logé dans une ouverture 4, la tête 22 du dispositif fait saillie de l'entrée 41 de l'ouverture et la pointe 21 du dispositif fait saillie en dehors du corps principal 3.

Les ouvertures 4 du corps principal 3 peuvent être réparties en plusieurs rangées, par exemple deux rangées R1 et R2, parallèles à la courbe génératrice 9. Les ouvertures d'une même rangée sont configurées pour que deux sorties 42 voisines sont séparées d'un pas 25 constant.

La figure 7 représente schématiquement un corps principal 3 et illustre le cas où les ouvertures sont réparties selon deux rangées R1 et R2.

Le pas 25 est typiquement compris entre 2 cm et 3cm. Les deux rangées sont séparées, selon la direction latérale L, d'une distance typiquement comprise entre 2 cm et 3cm. Les rangées sont décalées d'environ un demi-pas, c'est-à-dire une distance deux fois plus faible que le pas 25, entre elles.

Les ouvertures 4 peuvent être toutes identiques notamment si les dispositifs d'incision sont tous identiques.

Optionnellement, chaque élément 15 comprend une des ouvertures 4 et un des dispositifs 2, les éléments présentant une forme identique et pour chaque couple d'éléments adjacents les éléments 15 présentent des sens d'orientation opposés. Tous les éléments présentent la même forme, et deux éléments adjacents sont disposés tête bèche, le premier selon un premier sens de la direction latérale et le second selon le deuxième sens opposé au premier sens. Dans cette option, l'élément selon le premier sens présent une forme dans la région de la face cylindrique qui est complémentaire de la forme de l'élément adjacent orienté lui selon le deuxième sens.

Si les éléments présentent tous la même forme, il est possible de limiter les couts de fabrication.

Si de plus, le nombre d'éléments compris dans le corps principal est pair, il est possible de réaliser un appareil dans lequel les deux branches sont de forme identique.

### Butoir et Paroi de butée

L'appareil peut, dans une deuxième option dite option des butées, présenter les caractéristiques suivantes : chaque élément comprend :
- un butoir 35 configuré pour couvrir, lorsque l'appareil est dans la configuration d'incision, une tête 22 d'un premier dispositif 2 d'un premier élément adjacent, le butoir 35 étant en contact avec le premier dispositif, et/ou
- une paroi de butée 37 configurée pour, lorsque l'appareil est dans la configuration, être en contact avec une tête (2 d'un deuxième dispositif d'un deuxième élément adjacent différent du premier élément adjacent.

La première option des flancs complémentaires et la deuxième option des butées peuvent être mises en œuvre l'une sans l'autre ou être mises en œuvre de manière combinée. La combinaison des deux options est représentée aux figures 6 et 7.

Chaque élément 15 comprend une ouverture 4. Les ouvertures sont globalement réparties en deux rangées parallèles à la courbe génératrice 9 et séparées selon la direction latérale. Dans chaque rangée, les ouvertures sont régulièrement réparties le long de la courbe génératrice avec un pas constant, les rangées étant décalées d'environ un demi-pas.

Un demi-pas correspond à la distance séparant les axes de deux liaisons pivot adjacentes.

Deux ouvertures de deux éléments adjacents ne sont pas dans le prolongement l'une de l'autre selon la courbe génératrice 9, elles sont séparées par une distance non nulle selon la direction axiale ou latérale L.

Chaque ouverture comprend, côté intérieur, une entrée 41 par laquelle on fait entrer un dispositif d'incision cutanée.

L'élément comprend un butoir 35 et une paroi de butée 37 qui sont alignés selon la courbe génératrice avec l'ouverture d'un élément adjacent.

Chaque élément comprend d'une part une ouverture et d'autre part un butoir et une paroi de butée séparés par une distance non nulle selon la direction latérale. Cette distance est la distance selon la direction latérale séparant les deux rangées parallèles d'ouvertures.

Pour un élément donné :
- son ouverture est alignée selon la courbe génératrice avec le butoir et la paroi de butée de l'élément adjacent, et
- son butoir et sa paroi de butée sont alignés selon la courbe génératrice avec l'ouverture de l'élément adjacent.

Un butoir 35 présente un creux 36 de forme complémentaire à une tête 22 du dispositif d'incision. La forme est notamment complémentaire d'une partie supérieure de la tête et d'une partie de la face de la tête.

Lorsqu'une ouverture loge un dispositif d'incision, la tête du dispositif fait saillie de l'entrée de l'ouverture.

Il est possible de faire tourner l'élément portant le dispositif par rapport à un élément adjacent, jusqu'à un agencement limite où le butoir de l'élément adjacent vient couvrir au contact la tête du dispositif de sorte que la tête est logée dans le creux de forme complémentaire. La tête est alors en contact avec le butoir selon sa partie supérieure et une de ses faces. Dans cet agencement limite, le butoir peut être séparé d'une distance d'environ 1 millimètre de la tête du dispositif. Dans tous les cas cependant ; le butoir couvre la tête, de sorte que la tête ne peut quitter son ouverture.

Une paroi de butée présente une surface plane qui peut être en saillie de l'élément.

Il est possible de faire tourner l'élément portant le dispositif par rapport à un élément adjacent, jusqu'à un agencement limite où la paroi de butée de l'élément adjacent vient au contact d'une face de la tête du dispositif de sorte à appuyer contre cette face. La tête est alors en contact avec la paroi de butée selon une de ses faces.

Pour un élément donné :
- le butoir ne peut couvrir qu'un premier dispositif logé dans un premier élément adjacent, le butoir présente une orientation selon la courbe génératrice 9 qui est l'orientation de l'élément donné vers le premier élément adjacent,
- la paroi de butée ne peut entrer en contact qu'avec un deuxième dispositif logé dans un deuxième élément adjacent différent du premier élément adjacent, la paroi de butée présente une orientation selon la courbe génératrice 9 différente de l'orientation du butoir, à savoir l'orientation de l'élément donné vers le deuxième élément adjacent.

Le butoir et la paroi de butée sont orientés selon des sens différents de la courbe génératrice.

Lorsque tous les éléments présentent une forme identique et que les éléments adjacents sont disposés deux à deux selon des sens différents de la direction latérale, les dispositifs logés dans les éléments en extrémité de la juxtaposition ne peuvent pas être couverts par un butoir d'un élément adjacent ou alternativement ne peuvent pas être mis en contact avec une paroi de butée d'un élément adjacent.

Dans ce cas, il est possible d'ajouter selon le cas un butoir ou une paroi de butée à chacune des branches pour remplir ce rôle. Chaque branche comprend :
- soit un butoir présentant une forme complémentaire à une tête d'un dispositif d'incision cutanée, et dans un agencement limite entre une branche et un élément adjacent portant un dispositif, le butoir de la branche vient couvrir au contact la tête du dispositif de sorte que la tête est logée dans le creux de forme complémentaire,
- soit une paroi de butée contre laquelle on peut appuyer une tête d'un dispositif d'incision cutanée, et dans un agencement limite entre une branche et un élément adjacent portant un dispositif, la paroi de butée vient au contact d'une face de la tête du dispositif.

Il est ainsi possible de garantir qu'un dispositif quelle que soit l'ouverture dans laquelle il est logé est couvert par un butoir et maintenu contre une paroi de butée.

Dans le cas d'une combinaison de la première et de la deuxième option :
- la paroi de butée peut être parallèle à une surface de l'élément, telle qu'on l'a définie précédemment,
- l'agencement limite peut être obtenu lorsque les surfaces des éléments en regard l'une de l'autre entrent en contact,
- chaque élément 15 est configuré pour que les surfaces de contacts soient situées entre d'une part le butoir et la paroi de butée et d'autre part la face extérieure.

Dans le cas de la deuxième option des butées, la configuration d'incision correspond à la situation où chaque dispositif est couvert par un butoir et maintenu contre une paroi de butée. Le dispositif est ainsi retenu par la butée dans l'ouverture, le butoir et la paroi de butée. Dans cette situation chaque dispositif logé dans l'appareil est dans une position stable et contrôlée par rapport au corps principal.

### Configuration de retrait

Lorsque l'appareil comprend des dispositifs 2 logés dans des ouvertures 4 du corps et portant les pointes respectives, chaque ouverture 4 traversant le corps principal 3, l'appareil peut être apte à avoir une deuxième configuration, dite configuration de retrait dans laquelle chaque ouverture 4 est découverte de sorte à permettre un retrait d'un dispositif 2.

Lorsque l'appareil comprend une face cylindrique et des branches, la configuration de retrait de l'appareil correspond à un cas où la face cylindrique 7 plane et les deux branches 11,13 sont disjointes.

Une configuration de retrait, lorsque l'appareil comprend une face cylindrique 7 et des branches 11,13, est représentée à la figure 3.

Les deuxièmes extrémités des branches ne sont pas liées directement, les moyens de liaison amovibles sont désolidarisés. La courbe génératrice n'est pas contrainte pour prendre la forme d'un arc de cercle, et prend la forme d'un segment droit. La face cylindrique est plane.

Par rapport à la configuration d'incision, les éléments portant les dispositifs sont mis en rotation de sorte que les entrées des ouvertures sont éloignées les unes des autres. Typiquement l'angle de rotation entre deux éléments adjacents permettant de passer de la configuration d'incision à la configuration de retrait est compris entre 0,5° et 35°.

Comme les ouvertures traversent le corps principal perpendiculairement à la face cylindrique, les ouvertures des dispositifs sont orientées parallèlement les unes par rapport aux autres dans la configuration de retrait.

Dans la configuration de retrait, les entrées des ouvertures ne sont pas couvertes, c'est-à-dire que l'appareil ne présente aucune matière dans l'axe d'extension de l'ouverture au-dessus de l'entrée de l'ouverture, côté intérieur.

L'opérateur a ainsi accès à l'entrée pour retirer un dispositif ou remplacer un dispositif par un autre. En particulier, l'appareil est configuré pour que dans la configuration d'incision les branches ne s'étendent pas au-dessus des ouvertures. Dans cette configuration, les branches s'étendent, à partir de leur première extrémité, du côté intérieur de la face cylindrique en s'éloignant du centre de la face cylindrique.

Dans le cas de la première option des flancs complémentaires, cette configuration de retrait correspond à la situation où les surfaces situées en regard l'une de l'autre, telle qu'on les a définies plus haut, ne sont pas en contact.

Dans le cas de la deuxième option des butées, cette configuration de retrait correspond à la situation où chaque dispositif n'est pas couvert par un butoir ni maintenu contre une paroi de butée. Le butoir d'un élément est donc configuré pour couvrir un dispositif d'un élément adjacent dans la configuration d'incision et en plus pour que la rotation avec l'élément adjacent qui permet de passer à la configuration de retrait soit suffisante pour que le butoir ne couvre plus le dispositif.

### Utilisation de l'appareil pour application cutanée

L'appareil 1 tel qu'on l'a décrit plus haut comprend une pluralité de dispositifs que l'on peut préparer pour qu'ils contiennent chacun un réactif allergène particulier ou une solution vaccinale particulière.

Une telle structure dans sa configuration d'incision présente les pointes des dispositifs préparés qui sont orientées radialement vers l'extérieur selon une surface qui est convexe dans une direction longitudinale.

Un opérateur peut alors appliquer les pointes sur la peau d'une personne ou d'un animal en faisant rouler l'appareil dans la direction longitudinale. En faisant ce geste, les pointes s'approchent de la peau, piquent l'épiderme puis s'éloignent de la peau. Le caractère convexe permet de garantir que l'application des pointes s'effectue dans les mêmes conditions d'une pointe à une autre. L'application homogène et uniforme des différentes pointes permet que chaque pointe puisse effectivement inciser la peau et injecter le produit utile dans la peau.

Si la poignée présente une forme cylindrique qui s'étend selon la direction latérale, l'application de l'appareil est plus aisée pour l'opérateur. Le maintien de la poignée entre le pouce et l'index permet d'utiliser la rotation du poignet pour faire rouler l'appareil sur la peau du patient.

### Dévidoir

En complément de l'appareil, un ensemble de chargement de cette structure est proposé.

Dans premier mode de réalisation d'un ensemble de chargement, celui-ci comprend un appareil tel qu'on a pu la présenter précédemment et un dévidoir 50.

La figure 9 représente de manière schématique un dévidoir 50.

Le dévidoir 50 est une pièce rigide non déformable qui a essentiellement une forme de pavé droit ou parallélépipède rectangle. Le dévidoir présente une longueur 51, une largeur 52 et une hauteur 53, la longueur 51 étant significativement plus importante que la largeur 52 et la hauteur 53.

La longueur du dévidoir est supérieure à la longueur de la courbe génératrice du corps principal.

La largeur du dévidoir est supérieure à la largeur 5 du corps principal.

Le dévidoir comprend un logement dans une face supérieure du dévidoir.

La face supérieure est définie par une longueur et une largeur.

Le logement est centré dans la face supérieure et définit un volume dans le dévidoir qui a une forme essentiellement de pavé droit ou parallélépipède rectangle qui est plus petit dans chacune des trois dimensions que le dévidoir lui-même.

Le logement présente une longueur parallèle à la longueur du dévidoir qui est significativement plus importante que sa largeur et sa hauteur, respectivement parallèles à la largeur et la hauteur du dévidoir.

La longueur du logement est supérieure à la longueur de la courbe génératrice du corps principal.

La largeur du logement est à peine supérieure à la largeur 5 du corps principal. Le logement présente un fond parallèle à la face supérieure.

Sur les côtés du logement définis par une longueur une hauteur du logement, le logement comprend des cavités de centrage 57.

Le corps principal comprend lui des reliefs de centrage 59 de forme complémentaire aux cavités de centrage 57, les reliefs de centrage étant disposés le long de bords latéraux du corps principal, c'est-à-dire le long des bords aux deux extrémités de la largeur du corps principal.

Le dévidoir présente au niveau du fond du logement un creux qui lui aussi est centré par rapport à la face supérieure et lui aussi présente une forme essentiellement de pavé droit ou parallélépipède rectangle. Le creux est plus petit dans chacune des trois dimensions que le logement.

Le creux définit un fond parallèle à la face supérieure.

Le logement ne traverse pas le dévidoir de part en part.

Le dévidoir est configuré pour être posé sur une face inférieure opposée à la face supérieure, de sorte que le logement est ouvert vers le haut.

La hauteur du dévidoir est donc orientée selon la direction verticale.

Le creux est occupé par une lamelle 55, par exemple une lamelle de verre ou une lamelle en plastique, par exemple un plastique comprenant des Cyclo Oléfin (Co)-Polymères.

La lamelle est une plaque d'épaisseur comprise entre 2 et 8 millimètres.

La lamelle peut être retirée puis remise librement dans le creux. Des encoches dans le dévidoir peuvent être prévues sur les bords du creux afin de faciliter le placement ou le retrait de la lamelle dans le creux.

La lamelle comprend des trous 58, le nombre de trous est égal au nombre d'ouvertures comprises dans le corps principal. La disposition des trous correspond à la disposition des ouvertures du corps principal lorsque celui-ci est dans la configuration de retrait.

Le logement est configuré pour recevoir le corps principal de l'appareil en configuration de retrait.

En configuration de retrait le corps principal présente une courbe génératrice droite et une face cylindrique plane.

La longueur du logement et la largeur du logement sont ajustées pour permette d'y insérer le corps principal de sorte que
- la face cylindrique plane est insérée dans le logement et vient en regard, voire au contact, de la lamelle, et
- les reliefs de centrage sont insérés dans les cavités de centrage.

L'ajustement du logement est tel que les reliefs de centrage peuvent être inséré au fond des cavités de centrage.

Les trous de lamelle sont positionnés pour recevoir, lorsque l'appareil est inséré dans le dévidoir, les pointes des dispositifs qui font saillie de la face cylindrique plane.

La figure 10 représente schématiquement un appareil 1 en configuration de retrait reçue dans un dévidoir 50.

Les dimensions du logement sont ajustées aux dimensions du corps principal mais aussi aux dimensions des branches. Dans la configuration de retrait les premières extrémités des branches sont dans le prolongement du corps principal. Une partie des branches, du côté de leurs premières extrémités est insérée dans le logement en même que le corps principal.

Les dimensions du logement sont ajustées de sorte à réduire le jeu mécanique lorsque l'appareil est inséré dans le logement. Cela permet de stabiliser l'appareil dans le dévidoir.

A cet effet, le logement présente une hauteur suffisamment importante pour que l'ensemble des liaisons pivot de l'appareil se trouvent à l'intérieur du volume défini par le logement lorsque l'appareil est inséré dans le dévidoir.

De plus, il est possible de prévoir un ou deux reliefs de centrage par élément. Cela permet en insérant l'appareil dans le dévidoir de stabiliser la position de chaque liaison pivot.

L'insertion peut être facilitée en adaptant la forme du logement et de l'appareil. Par exemple le logement peut présenter des bords évasés vers l'extérieur, c'est-à-dire vers la face supérieure et en correspondance le corps principal et les branches peuvent présenter des bords évasés.

La forme du relief de centrage peut être adaptée à la présence des liaisons pivot. Par exemple le relief peut présenter deux surfaces courbes en regard des deux axes des liaisons pivot de l'élément. Chaque surface courbe définit un ou plusieurs centres de courbures qui se trouvent tous du côté du relief où se trouve l'axe de la liaison pivot. La cavité de pivot présente la forme complémentaire au relief de centrage.

Les mouvements du relief de centrage en rapport avec la liaison pivot induit un mouvement contre la cavité globalement dans le sens de la surface courbe, et ne vient pas s'opposer à l'insertion du relief dans la cavité. L'insertion de l'appareil dans le dévidoir est ainsi facilitée, et de plus la position de chaque liaison pivot est stabilisée.

L'appareil et le dévidoir peuvent comprendre en option des moyens de liaison amovibles pour se lier entre eux.

Les moyens de liaison sont des moyens de liaison amovible comme par exemple des aimants, des pièces en tissu auto-agrippant, des clips de fixation, etc...

Par exemple les branches peuvent comprendre sur la face de prise des moyens de liaisons amovibles et le dévidoir à la position correspondante dans le logement comprend des moyens de liaison complémentaire.

Cela permet en insérant l'appareil dans le dévidoir de stabiliser la position de chaque liaison pivot.

### Utilisation du dévidoir pour charger l'appareil

Dans un mode de réalisation, les dispositifs d'incision cutanée peuvent être rechargés dans l'appareil à l'aide d'un dévidoir tel qu'on a pu le présenter plus haut.

Dans une première étape, on prépare la lamelle en la nettoyant, puis en versant au moins un produit utile (un réactif allergène ou une solution vaccinale) dans au moins un trou de la lamelle.

Il est possible bien évidemment de verser un produit utile dans plusieurs trous voire dans tous les trous.

Les produits utiles versés peuvent être identiques ou différents d'un trou à un autre.

La lamelle peut être placée dans le creux avant ou après le versement des produits utiles.

L'appareil est adapté dans la configuration de retrait.

Si elle comprend des dispositifs d'incision vides, c'est-à-dire non chargés par un produit utile, alors il n'est pas utile de les remplacer.

Si elle comprend des dispositifs d'incision déjà utilisés, alors on peut les retirer de l'appareil et les remplacer par des dispositifs d'incision vides. Pour cela, il est possible, avant de verser un produit utile dans plusieurs trous voire dans tous les trous, de loger l'appareil dans la configuration de retrait pour en retirer les dispositifs d'incision déjà utilisés et les remplacer par des dispositifs vides.

Dans les deux cas, on obtient un appareil dans la configuration de retrait comprenant des dispositifs d'incision vides de produit utile.

On place ensuite l'appareil dans le logement. Les pointes des dispositifs d'incision se trouvent ainsi reçues dans les trous de la lamelle remplis de produit utile. Les pointes des dispositifs mises en contact avec le produit utile en aspirent une quantité. Les dispositifs d'incision se trouvent ainsi chacun chargés d'un produit utile.

Dans une dernière étape, l'appareil est adapté en configuration d'incision et prête à être employée pour une application sur la peau d'une personne ou d'un animal.

### Tête de chargement

Dans un deuxième mode de réalisation de l'ensemble, celui-ci comprend, en plus par rapport au premier mode de réalisation une tête de chargement.

Les figures 10 et 11 illustrent schématiquement le deuxième mode de réalisation. La tête de chargement 60 est une pièce rigide non déformable qui présente globalement la forme d'un pavé droit ou parallélépipède rectangle.

La tête de chargement présente une longueur 61, une largeur 62 et une hauteur 63, la hauteur 63 et la longueur 61 étant significativement plus importantes que la largeur 62.

La longueur et la hauteur de la tête de chargement ont à peu près la même valeur qui correspond à la longueur de la courbe génératrice du corps principal. La largeur de la tête de chargement est à peu près égale à la largeur du dévidoir. La tête de chargement est configurée pour être posée sur le dévidoir de sorte que la longueur, la largeur et la hauteur de la tête de chargement sont mesurées dans des directions respectivement parallèles à celles où on mesure la longueur, la largeur et la hauteur du dévidoir.

La tête de chargement comprend une face inférieure 65 définie par une longueur et une largeur configurée pour recouvrir le dévidoir, et une face supérieure 64 à l'opposé de cette face inférieure.

La tête de chargement est configurée pour couvrir au contact le corps principal de l'appareil en configuration de retrait quand il est reçu dans le logement, la face inférieure étant en appui sur des parois latérales du dévidoir, c'est-à-dire des parois du dévidoir définis par une hauteur et une longueur du dévidoir.

La hauteur de la tête de chargement est donc orientée selon la direction verticale.

La face inférieure de la tête de chargement peut présenter un relief adapté à un relief du corps principal côté intérieur en configuration de retrait. Dans le cas de la première et/ou de la deuxième option, le relief du corps principal côté intérieur peut notamment être défini par les surfaces en regard et/ou les butoirs et les parois de butées. Dans ce cas, la face intérieure de la tête de chargement est creusée de reliefs pour ne pas être bloqué par ces éléments.

La tête de chargement peut comprendre en option des épaulements situés en en saillie de bords latéraux de la tête de chargement, côté face intérieure, les bords latéraux étant définis par une longueur et une hauteur de la tête de chargement. Ces épaulements sont configurés pour venir en appui sur des parois latérales du dévidoir, lorsque la tête de chargement couvre le corps principal de l'appareil en configuration de retrait quand il est reçu dans le logement.

Dans un plan horizontal, la position de la tête de chargement peut être fixée par une complémentarité du relief de la face intérieure de la tête avec le relief du corps principal côté intérieur en configuration de retrait.

La tête de chargement comprend des canaux de chargement configurés pour permettre le glissement d'un dispositif d'incision. Plus précisément, un dispositif peut glisser dans le canal lorsqu'il est inséré dans le canal dans la direction définie par la tête et la pointe du dispositif, la pointe étant insérée la première. Chaque canal traverse la tête de chargement depuis la face supérieure 64 jusqu'à la face inférieure 65.

Chaque canal comprend une entrée 67 évasée côté face supérieure et une sortie côté face inférieure.

L'entrée évasée permet une insertion simple d'un dispositif. En particulier un opérateur peut saisir un dispositif par sa tête, placer la pointe à l'intérieur du canal à travers l'entrée du canal puis lâcher la tête du dispositif pour le faire glisser dans le canal.

Lorsque la tête de chargement couvre le corps principal reçu dans le logement, chaque sortie de canal de chargement se trouve en regard d'une ouverture du corps principal. Si un dispositif glisse dans le canal, celui-ci sort de la tête de chargement et entre dans l'ouverture en correspondance du canal.

En option, les canaux de chargement présentent une forme d'hélice autour d'une direction d'extension du canal - de la face inférieure à la face supérieure - de sorte que le glissement d'un dispositif d'incision depuis une face supérieure jusqu'à une face inférieure fait tourner le dispositif d'un angle compris entre 5 et 90° autour de la direction d'extension.

Cette option permet d'ajuster l'agencement du dispositif par rapport au corps principal. En particulier si les dispositifs sont conditionnés en un groupe de dispositifs agencés selon une rangée, les entrées évasées peuvent correspondre à ce conditionnement et la forme d'hélice des canaux permet un agencement des dispositifs installés dans le corps principal, différent de l'agencement de conditionnement.

### Utilisation de la tête de chargement pour charger l'appareil

Dans un mode de réalisation présente plus haut, les dispositifs 2 d'incision peuvent être chargés ou rechargés dans l'appareil à l'aide d'un dévidoir. Il est possible de compléter ce procédé par une étape de couverture du corps principal reçu dans le logement par la tête de chargement.

Cette étape de couverture intervient si l'appareil comprend des dispositifs d'incision déjà utilisés et qu'un opérateur les a retirés de l'appareil.

Avant de les remplacer par des dispositifs d'incision vides, le corps principal reçu dans le logement est couvert par la tête de chargement.

On réalise ensuite une étape d'insertion d'un dispositif d'incision vide de produit utile dans une entrée d'un canal de chargement. Cette étape peut être répétée pour plusieurs dispositifs d'incision dans plusieurs canaux de chargement. L'appareil reçoit ainsi une pluralité de dispositifs d'incision vides de produit utile. Chaque pointe de dispositif se trouve alors en contact avec du produit utile présent dans un trou de la lamelle et se charge en produit utile.

La tête de chargement peut être retirée au-dessus de l'appareil, l'appareil peut être retiré du dévidoir et agencée selon la configuration d'incision.

## Revendications

1. Appareil (1) pour injection dans la peau par incision superficielle comprenant :
- un corps principal (3), et
- des pointes (21) pour incision de la peau s'étendant en saillie du corps (3), l'appareil (1) étant apte à avoir une configuration dans laquelle :
- les pointes (21) définissent une surface intégralement courbe, la surface étant convexe dans une direction longitudinale, et
- un centre de courbure d'une section de la surface dans un plan longitudinal parallèle à la direction longitudinale et le corps sont d'un même côté de la surface,
l'appareil étant **caractérisé en ce qu'**il comprend deux branches (11, 13) disposées à deux extrémités longitudinales du corps principal (3), les deux branches (11, 13) étant directement liées l'une à l'autre lorsque l'appareil est dans la configuration de sorte à former une poignée (27), le corps principal (3) et la poignée (27) étant d'un même côté de la surface.

2. Appareil selon la revendication 1 dans lequel le corps principal comprend une face (7), les pointes (21) s'étendant en saillie de la face (7), la face (7) définissant lorsque l'appareil est dans la configuration un rayon de courbure moyen dans le plan longitudinal, le rayon de courbure moyen étant compris entre 7 et 20 centimètres.

3. Appareil selon la revendication 1 dans lequel la poignée (27) présente une forme cylindrique définie par une direction axiale (L) perpendiculaire à la direction longitudinale.

4. Appareil selon l'une des revendications 2 à 3 dans leur dépendance à la revendication 2, dans lequel, lorsque l'appareil est dans la configuration, la surface et la face sont concaves dans une direction axiale perpendiculaire à la direction longitudinale, la surface étant située entre un centre de courbure d'une section de la surface dans un plan axial parallèle à la direction axiale et le corps.

5. Appareil selon la revendication 2 à 3, dans lequel la face (7) est cylindrique, la face cylindrique étant définie par une direction axiale (L) perpendiculaire à la direction longitudinale.

6. Appareil selon la revendication 5, dans lequel la face (7), dans lequel le corps principal (3) comprend des éléments (15) liés deux à deux et juxtaposés dans un plan normal à la direction axiale (L), deux des éléments (15) étant liés chacun à une des branches (11, 13), chaque liaison (17, 29) entre deux éléments (15) adjacents ou entre un élément (15) et une branche (11, 13) permettant une rotation autour d'un axe parallèle à la direction axiale (L).

7. Appareil selon la revendication 6 dans lequel chaque élément (15) présente un flanc (31) en regard de chaque élément adjacent ou d'une branche adjacente, chaque branche présente un flanc (33) en regard d'un élément adjacent, de sorte que les flancs (31, 33) de deux éléments adjacents ou d'un élément et d'une branche adjacents sont complémentaires et, lorsque l'appareil est dans la configuration en contact l'un avec l'autre.

8. Appareil selon la revendication 7 dans lequel les flancs (31, 33) définissent, lorsque l'appareil est dans la configuration, un plan passant par un centre de courbure de la surface et par un axe de la liaison (17) liant les deux éléments ou l'élément et la branche.

9. Appareil selon l'une des revendications 6 à 8 comprenant des dispositifs (2) logés dans des ouvertures (4) du corps principal (3) et portant les pointes respectives, chaque ouverture (4) traversant le corps principal (3), chaque élément (15) comprenant une des ouvertures (4) et un des dispositifs (2), les éléments (15) présentant une forme identique et pour chaque couple d'éléments adjacents les éléments (15) présentant des sens d'orientation opposés.

10. Appareil selon la revendication 9, dans lequel chaque élément (15) comprend :
- un butoir (35) configuré pour couvrir, lorsque l'appareil est dans la configuration, une tête (22) d'un premier dispositif (2) d'un premier élément adjacent, le butoir (35) étant en contact avec le premier dispositif, ou
- une paroi de butée (37) configurée pour, lorsque l'appareil est dans la configuration, être en contact avec une tête (22) d'un deuxième dispositif d'un deuxième élément adjacent, ou
- un butoir (35) configuré pour couvrir, lorsque l'appareil est dans la configuration, une tête (22) d'un premier dispositif (2) d'un premier élément adjacent, le butoir (35) étant en contact avec le premier dispositif, et une paroi de butée (37) configurée pour, lorsque l'appareil est dans la configuration, être en contact avec une tête (22) d'un deuxième dispositif d'un deuxième élément adjacent différent du premier élément adjacent.

11. Appareil selon l'une des revendications précédentes comprenant des dispositifs (2) logés dans des ouvertures (4) du corps et portant les pointes respectives, chaque ouverture (4) traversant le corps principal (3), la configuration étant une configuration d'incision, l'appareil étant apte à avoir une configuration de retrait dans laquelle chaque ouverture (4) est découverte de sorte à permettre un retrait d'un dispositif (2).

12. Ensemble de chargement comprenant un appareil selon l'une des revendications précédentes, et un dévidoir (50), le dévidoir (50) comprenant un logement et une lamelle (55) agencée au fond du logement, le logement présentant des cavités (57) et la lamelle (55) comprenant des trous (58), le corps principal (3) comprenant des reliefs de centrage (59) de forme complémentaire aux cavités (57) et disposés le long de bords latéraux du corps principal, le logement étant configuré pour recevoir le corps principal (3) de l'appareil (1) de sorte que les reliefs (59) s'insèrent dans les cavités (57), et que les trous (58) reçoivent les pointes (21).

13. Ensemble selon la revendication 12, l'appareil étant un appareil selon la revendication 10 et la revendication 11, l'ensemble comprenant une tête (60), la tête (60) comprenant des canaux configurés pour permettre un glissement d'un dispositif d'incision (2), chaque canal traversant la tête (60) depuis une entrée évasée (67) dans une face supérieure (64) de la tête jusqu'à une sortie dans une face inférieure (65) de la tête, la tête (60) étant configurée pour couvrir le corps principal (3) de l'appareil (1) lorsque le corps principal (3) en configuration de retrait est reçu dans le logement, la face inférieure (65) étant en appui sur des parois latérales du dévidoir, chaque canal de chargement présentant une sortie en regard d'une ouverture (4) de sorte qu'un dispositif (2) glissant dans le canal passe dans l'ouverture (4).

14. Ensemble selon la revendication 13 dans lequel les canaux présentent une forme d'hélice autour d'une direction d'extension du canal, de sorte que le glissement d'un dispositif d'incision (2) depuis la face supérieure (64) jusqu'à la face inférieure (65) fait tourner le dispositif d'un angle de 90° autour de la direction d'extension.

15. Procédé d'incision superficielle cutanée dans lequel on fait rouler sur la peau d'un patient un appareil portant des pointes (21) d'incision de la peau en mettant les pointes successivement en contact avec la peau, l'appareil étant un appareil selon l'une des revendications 1 à 11, le procédé comprenant une étape d'adaptation de l'appareil (1) dans la configuration avant de le faire rouler sur la peau.

16. Procédé de préparation de l'appareil d'un ensemble selon la revendication 12, le procédé comprenant les étapes suivantes :
- nettoyage de la lamelle (55),
- versement d'un produit utile dans un trou (58) de la lamelle,
- réception de l'appareil (1) dans le logement.

17. Procédé selon la revendication 16 de préparation de l'appareil d'un ensemble selon la revendication 13 comprenant en outre une étape de couverture du corps principal (3) reçu dans le logement par la tête de chargement (60) et une étape d'insertion d'un dispositif d'incision (2) vide de produit utile dans une entrée (57) d'un canal de chargement.

## Patentansprüche

1. Gerät (1) zur Injektion in die Haut durch oberflächliches Einschneiden, umfassend:
- einen Hauptkörper (3) und
- Spitzen (21) zum Einschneiden der Haut, die sich vom Körper (3) vorstehend erstrecken,
wobei das Gerät (1) imstande ist, eine Konfiguration zu haben, in der:
- die Spitzen (21) eine vollständig gekrümmte Oberfläche definieren, wobei die Oberfläche in einer Längsrichtung konvex ist, und
- ein Krümmungsmittelpunkt eines Abschnitts der Oberfläche in einer zur Längsrichtung parallelen Längsebene und der Körper auf derselben Seite der Oberfläche sind,
wobei das Gerät **dadurch gekennzeichnet ist, dass** es zwei Schenkel (11, 13) umfasst, die an zwei Längsenden des Hauptkörpers (3) angeordnet sind, wobei die beiden Schenkel (11, 13) direkt miteinander verbunden sind, wenn das Gerät in der Konfiguration ist, so dass ein Griff (27) gebildet wird, wobei der Hauptkörper (3) und der Griff (27) auf derselben Seite der Oberfläche sind.

2. Gerät nach Anspruch 1, wobei der Hauptkörper eine Fläche (7) aufweist, wobei sich die Spitzen (21) von der Fläche (7) vorstehend erstrecken, wobei die Fläche (7), wenn das Gerät in der Konfiguration ist, einen mittleren Krümmungsradius in der Längsebene definiert, wobei der mittlere Krümmungsradius zwischen 7 und 20 Zentimetern liegt.

3. Gerät nach Anspruch 1, wobei der Griff (27) eine zylindrische Form aufweist, die durch eine zur Längsrichtung senkrechte axiale Richtung (L) definiert wird

4. Gerät nach einem der Ansprüche 2 bis 3 in ihrer Abhängigkeit von Anspruch 2, wobei, wenn das Gerät in der Konfiguration ist, die Oberfläche und die Fläche in einer zur Längsrichtung senkrechten axialen Richtung konkav sind, wobei sich die Oberfläche zwischen einem Krümmungsmittelpunkt eines Abschnitts der Oberfläche in einer zur axialen Richtung parallelen Axialebene und dem Körper befindet.

5. Gerät nach Anspruch 2 bis 3, wobei die Fläche (7) zylindrisch ist, wobei die zylindrische Fläche durch eine zur Längsrichtung senkrechte axiale Richtung (L) definiert wird.

6. Gerät nach Anspruch 5, wobei die Fläche (7), in der der Hauptkörper (3) Elemente (15) umfasst, die paarweise miteinander verbunden und in einer zur axialen Richtung (L) normalen Ebene nebeneinander angeordnet sind, wobei zwei der Elemente (15) jeweils mit einem der Schenkel (11, 13) verbunden sind, wobei jede Verbindung (17, 29) zwischen zwei benachbarten Elementen (15) oder zwischen einem Element (15) und einem Schenkel (11, 13) eine Drehung um eine zur axialen Richtung (L) parallele Achse ermöglicht.

7. Gerät nach Anspruch 6, wobei jedes Element (15) eine Flanke (31) aufweist, die jedem benachbarten Element oder einem benachbarten Schenkel zugewandt ist, wobei jeder Schenkel eine Flanke (33) aufweist, die einem benachbarten Element zugewandt ist, so dass die Flanken (31, 33) zweier benachbarter Elemente oder eines Elements und eines benachbarten Schenkels komplementär und, wenn das Gerät in der Konfiguration ist, in Kontakt miteinander sind.

8. Gerät nach Anspruch 7, wobei die Flanken (31, 33), wenn das Gerät in der Konfiguration ist, eine Ebene definieren, die durch einen Krümmungsmittelpunkt der Oberfläche und durch eine Achse der Verbindung (17) verläuft, die die beiden Elemente oder das Element und den Schenkel verbindet.

9. Gerät nach einem der Ansprüche 6 bis 8, umfassend Vorrichtungen (2), die in Öffnungen (4) des Hauptkörpers (3) untergebracht sind und die jeweiligen Spitzen tragen, wobei jede Öffnung (4) den Hauptkörper (3) durchquert, wobei jedes Element (15) eine der Öffnungen (4) und eine der Vorrichtungen (2) umfasst, wobei die Elemente (15) eine identische Form aufweisen und für jedes Paar benachbarter Elemente die Elemente (15) entgegengesetzte Ausrichtungen aufweisen.

10. Gerät nach Anspruch 9, wobei jedes Element (15) umfasst:
- einen Anschlag (35), der ausgelegt ist, um, wenn das Gerät in der Konfiguration ist, einen Kopf (22) einer ersten Vorrichtung (2) eines ersten benachbarten Elements abzudecken, wobei der Anschlag (35) mit der ersten Vorrichtung in Kontakt ist, oder
- eine Anschlagwand (37), die ausgelegt ist, um, wenn das Gerät in der Konfiguration ist, mit einem Kopf (22) einer zweiten Vorrichtung eines zweiten benachbarten Elements in Kontakt zu sein, oder
- einen Anschlag (35), der ausgelegt ist, um, wenn das Gerät in der Konfiguration ist, einen Kopf (22) einer ersten Vorrichtung (2) eines ersten benachbarten Elements abzudecken, wobei der Anschlag (35) mit der ersten Vorrichtung in Kontakt ist, und eine Anschlagwand (37), die ausgelegt ist, um, wenn das Gerät in der Konfiguration ist, mit einem Kopf (22) einer zweiten Vorrichtung eines zweiten benachbarten Elements in Kontakt zu sein, das sich von dem ersten benachbarten Element unterscheidet.

11. Gerät nach einem der vorstehenden Ansprüche, umfassend Vorrichtungen (2), die in Öffnungen (4) des Körpers untergebracht sind und die jeweiligen Spitzen tragen, wobei jede Öffnung (4) den Hauptkörper (3) durchquert, wobei die Konfiguration eine Einschneidekonfiguration ist, wobei das Gerät imstande ist, eine Rückzugskonfiguration zu haben, in der jede Öffnung (4) freigelegt ist, um ein Zurückziehen einer Vorrichtung (2) zu ermöglichen.

12. Ladeanordnung, umfassend ein Gerät nach einem der vorstehenden Ansprüche und einen Spender (50), wobei der Spender (50) eine Aufnahme und eine Lamelle (55) umfasst, die am Boden der Aufnahme angeordnet ist, wobei die Aufnahme Hohlräume (57) aufweist und die Lamelle (55) Löcher (58) umfasst, wobei der Hauptkörper (3) Zentrierungsvorsprünge (59) aufweist, deren Form komplementär zu den Hohlräumen (57) ist und die entlang der Seitenkanten des Hauptkörpers angeordnet sind, wobei die Aufnahme ausgelegt ist, um den Hauptkörper (3) des Geräts (1) derart aufzunehmen, dass die Vorsprünge (59) in die Hohlräume (57) passen und dass die Löcher (58) die Spitzen (21) aufnehmen.

13. Anordnung nach Anspruch 12, wobei das Gerät ein Gerät nach Anspruch 10 und Anspruch 11 ist, wobei die Anordnung einen Kopf (60) umfasst, wobei der Kopf (60) Kanäle umfasst, die ausgelegt sind, um ein Gleiten einer Einschneidevorrichtung (2) zu gestatten, wobei jeder Kanal den Kopf (60) von einem aufgeweiteten Eingang (67) in einer oberen Fläche (64) des Kopfes bis zu einem Ausgang in einer unteren Fläche (65) des Kopfes durchquert, wobei der Kopf (60) ausgelegt ist, um den Hauptkörper (3) des Geräts (1) abzudecken, wenn der Hauptkörper (3) in der Rückzugskonfiguration in der Aufnahme aufgenommen ist, wobei die untere Fläche (65) auf Seitenwänden des Spenders aufliegt, wobei jeder Ladekanal einen Ausgang aufweist, der einer Öffnung (4) zugewandt ist, so dass eine in dem Kanal gleitende Vorrichtung (2) in die Öffnung (4) passt.

14. Anordnung nach Anspruch 13, wobei die Kanäle eine Spiralform um eine Erstreckungsrichtung des Kanals aufweisen, so dass das Gleiten einer Einschneidevorrichtung (2) von der oberen Fläche (64) zur unteren Fläche (65) bewirkt, dass die Vorrichtung in einem Winkel von 90° um die Erstreckungsrichtung gedreht wird.

15. Verfahren zum oberflächlichen Einschneiden der Haut, wobei ein Gerät mit Spitzen (21) zum Einschneiden der Haut über die Haut eines Patienten gerollt wird, wobei die Spitzen nacheinander mit der Haut in Kontakt gebracht werden, wobei das Gerät ein Gerät nach einem der Ansprüche 1 bis 11 ist, wobei das Verfahren vor dem Rollen über die Haut einen Schritt des Anpassens des Geräts (1) an die Konfiguration umfasst.

16. Verfahren zur Vorbereitung des Geräts einer Anordnung nach Anspruch 12, wobei das Verfahren die folgenden Schritte umfasst:
- Reinigen der Lamelle (55),
- Einfüllen eines nützlichen Produkts in ein Loch (58) der Lamelle,
- Aufnehmen des Geräts (1) in der Aufnahme.

17. Verfahren nach Anspruch 16 zur Vorbereitung des Geräts einer Anordnung nach Anspruch 13, das ferner einen Schritt des Abdeckens des in der Aufnahme aufgenommenen Hauptkörpers (3) durch den Ladekopf (60) und einen Schritt des Einsetzens einer Einschneidevorrichtung (2) ohne nützliches Produkt in einen Eingang (57) eines Ladekanals umfasst.

## Claims

1. An apparatus (1) for injection into the skin by superficial incision comprising:
- a main body (3), and
- tips (21) for incision of the skin protruding from the body (3);
the apparatus (1) being able to have a configuration in which:
- the tips (21) define an entirely curved surface, the surface being convex in a longitudinal direction, and
- a center of curvature of a section of the surface in a longitudinal plane parallel to the longitudinal direction and the body are on the same side of the surface, the apparatus being **characterized in that** the apparatus comprises two branches (11, 13) disposed at two longitudinal ends of the main body (3), the two branches (11, 13) being directly linked to each other when the apparatus is in the configuration so as to form a handle (27), the main body (3) and the handle (27) being on the same side of the surface.

2. The apparatus according to claim 1 wherein the main body comprises a face (7), the tips (21) protruding from the face (7), the face (7) defining when the apparatus is in the configuration, an average radius of curvature in the longitudinal plane, the average radius of curvature being comprised between 7 and 20 centimeters.

3. The apparatus according to claim 1 wherein the handle (27) has a cylindrical shape defined by an axial direction (L) perpendicular to the longitudinal direction.

4. The apparatus according to any of claims 2 to 3 when dependent on claim 2, wherein, when the apparatus is in the configuration, the surface and the face are concave in an axial direction perpendicular to the longitudinal direction, the surface being located between a center of curvature of a section of the surface in an axial plane parallel to the axial direction and the body.

5. The apparatus according to claim 2 to 3, wherein the face (7) is cylindrical, the cylindrical face being defined by an axial direction (L) perpendicular to the longitudinal direction.

6. The apparatus according to claim 5, wherein the main body (3) comprises elements (15) linked in pairs and juxtaposed in a plane normal to the axial direction (L), two of the elements (15) each being linked to one of the branches (11, 13), each connection (17, 29) between two adjacent elements (15) or between an element (15) and a branch (11, 13) allowing a rotation about an axis parallel to the axial direction (L).

7. The apparatus according to claim 6, wherein each element (15) has a flank (31) facing each adjacent element or an adjacent branch, each branch has a flank (33) facing an adjacent element, so that the flanks (31, 33) of two adjacent elements or of an adjacent element and a branch are complementary and in contact with each other, when the apparatus is in the configuration.

8. The apparatus according to claim 7 wherein the flanks (31, 33) define, when the apparatus is in the configuration, a plane passing through a center of curvature of the surface and through an axis of the connection (17) linking the two elements or the element and the branch.

9. The apparatus according to any of claims 6 to 8 comprising devices (2) housed in openings (4) of the main body (3) and carrying the respective tips, each opening (4) passing through the main body (3), each element (15) comprising one of the openings (4) and one of the devices (2), the elements (15) having an identical shape and for each pair of adjacent elements the elements (15) having opposite directions of orientation.

10. The apparatus according to claim 9, wherein each element (15) comprises:
- a stopper (35) configured to cover, when the apparatus is in the configuration, a head (22) of a first device (2) of a first adjacent element, the stopper (35) being in contact with the first device, or
- an abutment wall (37) configured, when the apparatus is in the configuration, to be in contact with a head (22) of a second device of a second adjacent element, or
- a stopper (35) configured to cover, when the apparatus is in the configuration, a head (22) of a first device (2) of a first adjacent element, the stopper (35) being in contact with the first device, and an abutment wall (37) configured, when the apparatus is in the configuration, to be in contact with a head (22) of a second device of a second adjacent element different from the first adjacent element.

11. The apparatus according to any of the preceding claims comprising devices (2) housed in openings (4) of the body and carrying the respective tips, each opening (4) passing through the main body (3), the configuration being an incision configuration, the apparatus being able to have a withdrawal configuration in which each opening (4) is uncovered so as to allow withdrawal of a device (2).

12. A loading assembly comprising an apparatus according to any of the preceding claims, and a dispenser (50), the dispenser (50) comprising a housing and a strip (55) arranged at the bottom of the housing, the housing having cavities (57) and the strip (55) comprising holes (58), the main body (3) comprising centering reliefs (59) of complementary shape to the cavities (57) and disposed along lateral edges of the main body, the housing being configured to receive the main body (3) of the apparatus (1) so that the reliefs (59) are inserted into the cavities (57), and so that the holes (58) receive the tips (21).

13. The assembly according to claim 12, the apparatus being an apparatus according to claim 10 and claim 11, the assembly comprising a head (60), the head (60) comprising channels configured to allow a sliding of an incision device (2), each channel passing through the head (60) from a flared inlet (67) in an upper face (64) of the head up to an outlet in a lower face (65) of the head, the head (60) being configured to cover the main body (3) of the apparatus (1) when the main body (3) in withdrawal configuration is received in the housing, the lower face (65) bearing on lateral walls of the dispenser, each loading channel having an outlet facing an opening (4) so that a device (2) sliding in the channel passes into the opening (4).

14. The assembly according to claim 13 wherein the channels have a helix shape around a direction of extension of the channel, so that the sliding of an incision device (2) from the upper face (64) up to the lower face (65) rotates the device by an angle of 90° around the direction of extension.

15. A superficial skin incision method wherein an apparatus carrying skin incision tips (21) is rolled over the skin of a patient by placing the tips successively in contact with the skin, the apparatus being an apparatus according to any of claims 1 to 11, the method comprising a step of adapting the apparatus (1) in the configuration before rolling it over the skin.

16. A method for preparing the apparatus of an assembly according to claim 12, the process comprising the following steps:
- cleaning the strip (55),
- pouring a useful product into a hole (58) of the strip,
- receving (1) the apparatus in the housing.

17. The method according to claim 16 for preparing the apparatus of an assembly according to claim 13 further comprising a step of covering the main body (3) received in the housing by the loading head (60) and an step of inserting an incision device (2) empty of useful product into an inlet (57) of a loading channel.
